# EUROPEAN PATENT SPECIFICATION

(11) **EP 2 604 298 B1**
(45) Date of publication and mention of the grant of the patent: **30.03.2016**
(21) Application number: 11386025.8
(22) Date of filing: 15.12.2011
(51) Int. Cl.: A61L 12/08, C11D 3/382, C11D 3/48, A01N 65/12

(54) **Multipurpose solutions for contact lens care comprising chamomile**
Mehrzweck-Lösungen zur Pflege von Kontaktlinsen, die Kamille enthalten
Solutions multifonctionnelles pour l'entretien de lentilles de contact contenant de la camomille

(43) Date of publication of application: 19.06.2013
(73) Proprietor: DEMO SA Pharmaceutical Industry, 145 68 Krioneri Attikis (GR)
(72) Inventor: Demos, Dimitris, 14568, Krioneri, Attica (GR)

(56) References cited:
- EP-A1- 1 666 027
- WO-A2-2006/057755
- DE-A1-102007 025 561
- JP-A- 2002 328 344
- JP-A- 2006 163 008

## Description

### Technical field

The present invention belongs to the field of contact lens care. In particular, it concerns multipurpose solutions used for cleaning, disinfecting, storing and rinsing contact lenses, which contain chamomile extract in their composition, at a concentration ranging from 0.010 to 0.050 mg/ml, so that adverse effects on the eyes caused by such solutions are practically eliminated, or at least substantially alleviated.

### General background

Contact lenses need to be treated with an appropriate solution on a regular basis because a variety of microbes and materials have the tendency to accumulate on the lenses. Specifically, tear film and debris consisting of proteins, carbohydrates, lipids and related organic matter have the tendency to deposit and build up on lens surfaces. As part of a routine cleaning system, contact lenses must be cleaned to remove these tear film deposits and debris, otherwise the wettability and optical clarity of the lenses are substantially reduced causing discomfort.

Various products are used to this effect (e.g. daily cleaners, hydrogen peroxide solutions, enzyme tablets etc.), but multipurpose contact lenses solutions (MPSs) are most popular for cleaning and disinfecting contact lenses, due to their multidimensional role and practicality. An MPS should provide effective removal of organic debris, ensure disinfection from microbes, enhance comfort of the lens wearer and retain the ocular hygiene and safety without causing any alteration in the structural characteristics of the contact lens (shrinkage or swelling) - the latter phenomenon regarding solely soft contact lenses.

MPSs generally comprise an antimicrobial agent for disinfecting and preservative action, a surfactant, a chelator with supplementary or synergistic antibiotic properties, wetting agents, a buffering system to maintain the pH of the solution, viscosity inducing compounds, tonicity agents and agents able to efficiently remove any material deposited on the lenses.

An example of a commercial multipurpose solution is Renu^{®} Multi-Purpose Solution (Bausch & Lomb), its composition being as follows:

| **Component** | **Function** |
|---|---|
| Polyhexamethylene biguanide (PHMB or DYMED^{®}) | Disinfectant, preservative |
| Boric acid | pH adjustment (buffer) |
| Sodium Borate | |
| Disodium edetate (Na²⁻ EDTA) | Chelating agent |
| Poloxamine | Non-ionic surfactant (removes proteins and lipids, i.e. build-up of tear film deposits and surface debris) |
| Sodium chloride (NaCl) | Tonicity agent |
| Purified water | Solvent |

A similar product by Bausch & Lomb is Renu MultiPlus^{®} Multi-Purpose Solution, which has the same composition, containing only hydroxyalkylphosphonate (HYDRANATE^{®}) in addition. This phosphonic compound removes proteins deposited on the contact lenses.

Despite their practicality and popularity, multipurpose solutions are not devoid of adverse effects, In the case of Renu^{®} and Renu MultiPlus^{®} these may be (according to the user leaflet): eyes sting, burn or itch (irritation), comfort is less than when lens was first placed on the eye, feeling of something in the eye (foreign body, scratched area), excessive watering (tearing) of the eye, unusual eye secretions, redness of the eye, reduced sharpness of vision (poor visual acuity), blurred vision, rainbows or halos around objects, sensitivity to light (photophobia), or dry eyes.

### Summary of the invention

The present invention aims at substantially alleviating the adverse reactions caused by multipurpose solutions for contact lens care, practically eliminating such reactions, via the addition of chamomile extract in their composition, at a concentration ranging from 0.010 to 0.050 mg/ml.

The use of chamomile extract in MPSs not only ensures a prolonged feeling of comfort for the lens user, thus allowing for the trouble-free, longer than usual wearing of contact lenses, but also improves the cleaning and disinfecting action of the MPS.

Another object of the present invention is to provide an MPS comprising chamomile extract which is effective, easy to use and can be prepared with conventional and inexpensive manufacturing methods, such as a simple blending of the ingredients.

### Commentary on the state of the art

Dried chamomile flower is an age-old medicinal drug known in ancient Egypt, Greece and Rome. Chamomile has been used for centuries in teas as a mild, relaxing sleep aid, in the treatment of fever, cold, stomach ailments, and as an anti-inflammatory, to name but a few of its therapeutic uses. Extensive scientific research over the past 20 years has confirmed many of the traditional uses of chamomile and established pharmacological mechanisms for the plant's therapeutic activities, including its antiseptic, antispasmodic, antipyretic, antibacterial, antifungal and antiallergic activity. Recent and on-going research has identified chamomile's specific anti-inflammatory, antiallergic, antiseptic and sedative properties, validating its long-held reputation.

Among its diverse applications, chamomile can assist in treating eye inflammation and infection. Cooled chamomile tea has been and is still used in compresses to soothe tired, irritated eyes and it may even help in the treatment of conjunctivitis.

However, the use of chamomile extract in aqueous multipurpose solutions for contact lens care at a concentration ranging from 0.010 to 0.050 mg/ml has not been proposed until now.

EP1666027 (Senju Pharmaceutical Co.) discloses an ophthalmic composition for contact lens, wherein the composition is an oil-in-water type emulsion comprising a refreshing agent and/or chlorobutanol together with an oil and an emulsifying agent in water. The refreshing agent may be "chamomile perfume", among others. The problem of the adsorption of refreshing agents by contact lenses has also been acknowledged by EP 1666027, but it is solved in a different manner, i.e. by adding an oil and an emulsifying agent to form an oil-in-water emulsion.

JP2002328344 (Fancl Corp.) discloses a cleaning agent composition for contact lenses comprising one or more oily components and one more surfactants (non-ionic, anionic, cationic, amphoteric), characterized in that it contains no water. The oily component is used as a base (a solvent), instead of water, and may be - among others - a chamomillae flos oil or an Anthemis nobilis oil, its concentration ranging from 20 to 99.99%.

JP2006163008 (Asahi Kasei Chemicals Corp.) discloses a contact lens cleaning agent containing a multi-chain and multi-hydrophilic group type compound having long-chain hydrophobic group and hydrophilic groups in one molecule. The contact lens cleaning agent further comprises a moisturizing component, which may be an extract of flos chamomillae, among others, at a concentration ranging from 3 to 50% w/w. Concentrations lower than 3% w/w, where the range claimed by the present application lies, should be avoided as they are deemed insufficient ("may not be sufficient as the stability effect of an enzyme").

WO2006057755 (Johnson & Johnson), titled "Compositions containing Cotinus coggygria extract and use thereof on mucosal tissues", refers to compositions which stimulate the production of mucus from mucosal tissues (especially vaginal and oral mucosal tissue) and enhance the elasticity or structural integrity of the skin and mucosal tissues, retarding the effects of aging. The Cotinus coggygria extract inhibits the activity of both elastase - thus protecting elastin fibers from damage - and trypsin - thus protecting tissues from proteolytic damage and degradation.
Besides the Cotinus Coggygria extract, said compositions may also contain other plant extracts, derived from Arctostaphylos uva-ursi, Matricaria chamomilla, Thymus vulgaris, Thymus serpyllum and/or Matricaria recutita. No reference whatsoever was found in the description with regard to the properties attributed to the compositions by any of these plant extracts, let alone Matricaria recutita.
There is a mere statement in the description that the compositions may be administered to the skin or mucosal tissue by using the hands or an applicator such as a wipe, tube, roller, spray, vaginal applicator, patch, tampon, toothbrush, suppository, inhaler, nasal spray, nasal dropper, eye dropper, contact lens, candy and gums. This, however, may not be considered as a specific disclosure of a contact lens cleaning solution containing chamomile.

Other prior art patent documents concern compositions / formulations for eye treatment which may comprise chamomile, but have nothing to do with contact lens care.

For example, US2004186405 (Biofarm) refers to a *pad* with a gel layer having cosmetic or therapeutic activity. The pad may be applied over an eye so as to lightly compress the eyelids and, if necessary, retained with a conventional plaster.
The pad consists of a flexible porous support, on at least one surface of which is applied a layer of gel comprising water, a dermatologically compatible polymer, a substance of plant origin comprising essential oils and aromatic extracts, and at least one dermatologically compatible component.
The preferred essential oils and aromatic extracts are chosen from oils and extracts of silver fir, star anice, bitter orange, sweet orange, benzoin absolute, bergamot, cajeput, Roman chamomile, white camphor, cinnamon leaves, carrot seeds, citron fruits, cypress, citronella, eucalyptus globulus etc.

Similarly, GB2007091 (Toko Yakuhin Kogyo KK) claims an *ophthalmic gel* comprising an aqueous solution of a carboxyvinyl polymer, a water-soluble basic substance and an ophthalmic drug admixed therewith, the gel having a pH of 5 to 8 and a viscosity of 1,000-100,000 cP at 20°C.

In example 3 of the description, a 1% aqueous solution of carboxyvinyl polymer was mixed with an aqueous solution of azulene (active component of chamomile flowers). To the mixture was added an aqueous solution of sodium hydroxide, with stirring, to give a gel composition having a pH of 7 and a viscosity of 4,000 cP.

### Detailed description of the invention and its preferred embodiments

New compositions for multipurpose solutions for contact lens care are proposed, intended at achieving the simultaneous cleaning, disinfecting, wetting and conditioning of the contact lenses, said compositions comprising chamomile extract at a concentration ranging from 0.010 to 0.050 mg/ml.

Besides chamomile, the multipurpose solutions of the present invention comprise surfactants and other cleaning agents for the removal of deposits and debris, wetting agents, buffering systems to maintain the pH of the solution, chelators with supplementary or synergistic antibiotic properties, antimicrobial agents for disinfecting and preservative action and tonicity agents. Some of the ingredients may possess multiple properties.

A suitable non-ionic surfactant consisting of a polyalkoxylated symmetrical block polymer of ethylene diamine, commonly known as Poloxamine and registered under the trade name Tetronic^{®} (BASF), has been found to be particularly effective in cleaning and wetting of soft contact lenses and has been chosen in the ophthalmic formulations of the present invention.

Among the principal components of multipurpose solutions is also included a complex forming agent that facilitates the removal of protein deposits from contact lenses. The preferred cleaning systems utilized in the present invention are sodium citrate or a mixture of phosphates (NaH₂PO₄ / Na₂HPO₄). In addition to their proven disinfecting activity, said compounds afford solutions with desired physicochemical properties, such as increased osmolality (e.g. in a range of 280-350 mOsm/kg and preferably about 320 mOsm/kg).

The pH of the formulations should be maintained in the range of 6.5-7.8, preferably 7.0-7.6. Buffers are formed by combining the weak bases mentioned above (sodium citrate, mixture of phosphates) with an acid such as boric acid.

In addition to buffers, sequestering agents (chelators) are present in the solutions, in order to bind metal ions which may accumulate on the contact lenses. Disodium edetate (Na₂-EDTA) is utilized to this end.

As for antimicrobial agents, the three most widely used ones at present in solutions for contact lens care arc polyhexamethylene biguanide (PHMB), alexidine and polyquaternium-1, which have proven effective in killing microbes associated with ocular infections and, at the same time, compatible with the ocular surface.

Finally, suitable tonicity agents used in the proposed formulations include sodium chloride and sorbitol. The tonicity agents are employed in such amount as to adjust the osmolality in the region of 280-350mOsm/kg.

Additional beneficial properties to the formulations of the present invention are endowed with the use of chamomile extract, wherein lies the major contribution of the present invention to the state of the art. Chamomile not only exerts a cleaning and disinfecting action itself, but, most importantly, it has a soothing effect and eliminates the feeling of dry eyes, irritation and other adverse reactions that contact lens wearers using multipurpose solutions are faced with. Chamomile has a long-lasting wetting effect and provides a feeling of ocular comfort, permitting the use of contact lenses throughout longer periods of time and under harsher conditions.

It should be noted that the term "chamomile extract" as used in this application is meant to include an extract derived from any of the plant species labeled as "chamomile" (all of the family Asteraceae), such as (mainly):
- Matricaria recutita (syn. M. chamomilla), German or blue chamomile,
- Anthemis nobilis (syn. Chamaemelum nobile), Roman chamomile,
but also (secondarily):
- Ormenis multicaulis (syn. Cladanthus mixtus,), Moroccan chamomile,
- Eriocephalus punctulatus, Cape chamomile,
- Matricaria discoidea, wild chamomile,
- Anthemis arvensis, scentless chamomile,
- Anthemis tinctoria, yellow chamomile or golden marguerite.

The concentration of chamomile extract in the MPS of the present invention ranges from 0.010 to 0.050 mg/ml, Preferably, it is about 0.025 mg/ml (or 0.0025% w/v).
Care should be taken that the concentration of chamomile extract does not exceed 0.100 mg/ml, because then contact lenses may be tinted by the extract, which may in turn alter their optical efficiency.

Several formulations with differences in qualitative and quantitative composition were developed and examined in order to conclude. The most preferred compositions are given in the following examples.

### Example 1

The MPS comprises sodium citrate as a cleaning agent and has the following composition:

| **Ingredient** | **mg/ml** | **Function** |
|---|---|---|
| **Poloxamine 1107** | 10.0 | Surfactant, wetting, cleaning agent |
| **Boric acid** | 6.4 | Antimicrobial enhancer, buffer component |
| **Sodium citrate** | 6.0 | Cleaning agent, protein remover, buffer component |
| **Disodium edetate (Na₂-EDTA)** | 1.0 | Chelating agent, antimicrobial enhancer |
| **Chamomile extract** | 0.025 | Soothing agent, enhancing comfort. Also cleaning, disinfecting, wetting agent |
| **Polyhexamethylene biguanide (PHMB or DYMED^{®})** | 0.001 | Disinfectant |
| **Sorbitol** | 10.0 | Tonicity agent |
| **Sodium chloride (NaCl)** | 3.5 | Tonicity agent |
| **NaOH / HCl** | q.s. for pH = 7.0-7.6 | pH adjusting agent |

### Example 2

The MPS comprises a mixture of phosphates (NaH₂PO₄ / Na₂HPO₄) as a cleaning agent and has the following composition:

| **Ingredient** | **mg/ml** | **Function** |
|---|---|---|
| **Poloxamine 1107** | 10.0 | Surfactant, wetting, cleaning agent |
| **Boric acid** | 5.0 | Antimicrobial enhancer, buffer component |
| **Na₂HPO₄** | 2.0 | Cleaning agent, protein remover, buffer |
| **NaH₂PO₄** | 0.08 | |
| **Disodium edetate (Na₂-EDTA)** | 1.0 | Chelating agent, antimicrobial enhancer |
| **Chamomile extract** | 0.025 | Soothing agent, enhancing comfort, Also cleaning, disinfecting, wetting agent |
| **Polyhexamethylene biguanide (PHMB or DYMED^{®}**) | 0.001 | Disinfectant |
| **Sodium chloride (NaCl)** | 6.0 | Tonicity agent |
| **NaOH / HCl** | q.s. for pH = 7.0-7.6 | pH adjusting agent |

Of course, the invention is not limited to the multipurpose solutions of the two examples. On the contrary, this application is intended to encompass other compositions as well, so long as they are covered by the scope of the claims.

## Claims

1. An aqueous multipurpose solution for cleaning, disinfecting, conditioning, storing and rinsing contact lenses, which comprises the following ingredients:
- a surfactant, for wetting the lenses and removing debris therefrom,
- a cleaning agent, for facilitating protein removal,
- a buffering system, for maintaining the pH of the solution,
- an antimicrobial agent, for disinfecting and preserving the lenses,
- a chelating agent, for binding metal ions,
- a tonicity agent, for adjusting the osmolality of the solution,
**characterized in that** it further comprises a chamomile extract at a concentration ranging from 0.010 to 0.050 mg/ml.

2. A multipurpose solution according to claim 1, **characterized in that** the chamomile extract is derived from one or more of the following plant species, all of the family Asteraceae, namely:
- Matricaria recutita or M. chamomilla, German or blue chamomile,
- Anthemis nobilis or Chamaemelum nobile, Roman chamomile,
- Ormenis multicaulis or Cladanthus mixtus, Moroccan chamomile,
- Eriocephalus punctulatus, Cape chamomile,
- Matricaria discoidea, wild chamomile,
- Anthemis arvensis, scentless chamomile,
- Anthemis tinctoria, yellow chamomile or golden marguerite.

3. A multipurpose solution according to claims 1 or 2, **characterized in that** the concentration of the chamomile extract is 0.025 mg/ml.

4. A multipurpose solution according to any preceding claim, wherein the surfactant is poloxamine.

5. A multipurpose solution according to any preceding claim, wherein the antimicrobial agent is polyhexamethylene biguanide, PHMB.

6. A multipurpose solution according to any preceding claim, wherein the chelating agent is disodium edetate, Na₂-EDTA.

7. A multipurpose solution according to any preceding claim, wherein the tonicity agent is sodium chloride and/or sorbitol.

8. A multipurpose solution according to any preceding claim, wherein the acidic component of the buffering system is boric acid.

9. A multipurpose solution according to any of claims 1 to 8, wherein the cleaning agent and alkaline component of the buffering system is sodium citrate.

10. A multipurpose solution according to claim 9, with the following composition:
| **Ingredient** | **mg/ml** |
|---|---|
| Poloxamine 1107 | 10.0 |
| Boric acid | 6.4 |
| Sodium citrate | 6.0 |
| Disodium edetate (Na₂-EDTA) | 1.0 |
| Chamomile extract | 0.025 |
| Polyhexamethylene biguanide (PHMB) | 0.001 |
| Sorbitol | 10.0 |
| Sodium chloride (NaCl) | 3.5 |
| NaOH/HCl | q.s. for pH= 7.0-7.6 |

11. A multipurpose solution according to any of claims 1 to 8, wherein the cleaning agent and alkaline component of the buffering system is a mixture of monobasic sodium phosphate, NaH₂PO₄ and dibasic sodium phosphate, Na₂HPO₄.

12. A multipurpose solution according to claim 11, with the following composition:
| **Ingredient** | **mg/ml** |
|---|---|
| Poloxamine 1107 | 10.0 |
| Boric acid | 5.0 |
| Na₂HPO₄ | 2.0 |
| NaH₂P0₄ | 0.08 |
| Disodium edetate (Na₂-EDTA) | 1.0 |
| Chamomile extract | 0.025 |
| Polyhexamethylene biguanide (PHMB) | 0.001 |
| Sodium chloride (NaCl) | 6.0 |
| NaOH / HCl | q.s. for pH = 7.0-7.6 |

## Patentansprüche

1. Wässrige Mehrzweck-Lösung zur Reinigung, Desinfektion, Konditionierung, Lagerhaltung und Spülung von Kontaktlinsen, die die folgenden Ingredienzien enthält:
- ein Tensid, zum Benetzen der Linsen und Entfernen von Fremdkörpern,
- ein Reinigungsmittel, zur Erleichterung der Proteinentfernung,
- ein Puffersystem, zum Behalten des pH-Wertes der Lösung,
- ein antimikrobielles Mittel, zur Desinfektion und Erhaltung der Linsen,
- einen Komplexbildner, zum Binden von Metallionen,
- ein Tonizitätsmittel, zum Einstellen der Osmolalität der Lösung,
**dadurch gekennzeichnet daß** sie auch ein Kamillenextrakt enthält, in einer Konzentration die von 0,010 bis 0,050 mg/ml reicht.

2. Mehrzweck-Lösung gemäß Anspruch 1, **dadurch gekennzeichnet daß** das Kamillenextrakt aus einer oder mehreren der folgenden Pflanzenarten, allen innerhalb der Familie Asteraceae, abgeleitet ist, nämlich aus:
- Matricaria recutita oder M. chamomilla, der Echten Kamille,
- Anthemis nobilis oder Chamaemelum nobile, der Römischen Kamille,
- Ormenis multicaulis oder Cladanthus mixtus, der Marokkanishen Kamille,
- Eriocephalus punctulatus, der Kap-Kamille,
- Matricaria discoidea, der wilden Kamille,
- Anthemis arvensis, der geruchlosen Kamille,
- Anthemis tinctoria, der gelben Kamille oder goldenen Margerite.

3. Mehrzweck-Lösung gemäß Anspruch 1 oder 2, **dadurch gekennzeichnet daß** die Konzentration von Kamillenextrakt 0,025 mg/ml ist.

4. Mehrzweck-Lösung gemäß einem der vorstehenden Ansprüche, worin das Tensid Poloxamin ist.

5. Mehrzweck-Lösung gemäß einem der vorstehenden Ansprüche, worin das antimikrobielle Mittel Polyhexamethylenbiguanid, PHMB, ist.

6. Mehrzweck-Lösung gemäß einem der vorstehenden Ansprüche, worin der Komplexbildner Dinatrium-ethylendiamin-tetraacetat, Na₂-EDTA, ist.

7. Mehrzweck-Lösung gemäß einem der vorstehenden Ansprüche, worin das Tonizitätsmittel Natriumchlorid und/oder Sorbitol ist.

8. Mehrzweck-Lösung gemäß einem der vorstehenden Ansprüche, worin die Säurekomponente des Puffersystems die Borsäure ist.

9. Mehrzweck-Lösung gemäß einem der Ansprüche 1 bis 8, worin das Reinigungsmittel und alkalische Komponente des Puffersystems das Natriumcitrat ist.

10. Mehrzweck-Lösung gemäß Anspruch 9, mit der folgenden Zusammensetzung:
| **Ingrediens** | **mg/ml** |
|---|---|
| Poloxamin 1107 | 10,0 |
| Borsäure | 6,4 |
| Natriumcitrat | 6,0 |
| Dinatrium-ethylendiamin-tetraacetat (Na₂-EDTA) | 1,0 |
| Kamillenextrakt | 0,025 |
| Polyhexamethylenbiguanid (PHMB) | 0,001 |
| Sorbitol | 10,0 |
| Natriumchlorid (NaCl) | 3,5 |
| NaOH / HCl | q.s. auf pH = 7,0-7,6 |

11. Mehrzweck-Lösung gemäß einem der Ansprüche 1 bis 8, worin das Reinigungsmittel und alkalische Komponente des Puffersystems ein Gemisch aus Mononatriumphosphat, NaH₂PO₄, und Dinatriumphosphat, Na₂HPO₄, ist.

12. Mehrzweck-Lösung gemäß Anspruch 11, mit der folgenden Zusammensetzung:
| **Ingrediens** | **mg/ml** |
|---|---|
| Poloxamin 1107 | 10,0 |
| Borsäure | 5,0 |
| Na₂HPO₄ | 2,0 |
| NaH₂PO₄ | 0,08 |
| Dinatrium-ethylendiamin-tetraacetat (Na₂-EDTA) | 1,0 |
| Kamillenextrakt | 0,025 |
| Polyhexamethylenbiguanid (PHMB) | 0,001 |
| Natriumchlorid (NaCl) | 6,0 |
| NaOH / HCl | q.s. auf pH = 7,0-7,6 |

## Revendications

1. Solution multifonctionelle aqueuse pour nettoyer, désinfecter, conditionner, entreposer et rincer des lentilles de contact, qui comprend les ingredients suivants:
- un agent tensioactif, pour mouiller les lentilles et enlever des débris de celles-ci,
- un agent de nettoyage, pour faciliter l'enlèvement des protéines,
- un système tampon, pour maintenir le pH de la solution,
- un agent antimicrobien, pour désinfecter et conserver les lentilles,
- un agent chélatant, pour fixer des ions métalliques,
- un agent de tonicité, pour ajuster l'osmolalité de la solution,
**caractérisée en ce qu'**elle comprend en outre un extrait de camomille dans une concentration allant de 0,010 à 0,050 mg/ml.

2. Solution multifonctionelle selon la revendication 1, **caractérisée en ce que** l'extrait de camomille est dérivé d'une ou de plusieurs des espèces de plantes suivantes, toutes de la famille Astéraceae, à savoir:
- Matricaria recutita ou M. chamomilla, camomille allemande,
- Anthemis nobilis ou Chamaemelum nobile, camomille romaine,
- Ormenis multicaulis ou Cladanthus mixtus, camomille du Maroc,
- Eriocephalus punctulatus, camomille du Cap,
- Matricaria discoidea, camomille sauvage,
- Anthemis arvensis, camomille sans parfum,
- Anthemis tinctoria, camomille jaune ou marguerite dorée.

3. Solution multifonctionelle selon la revendication 1 ou 2, **caractérisée en ce que** la concentration de l'extrait de camomille est 0,025 mg/ml.

4. Solution multifonctionelle selon l'une quelconque des revendications précédentes, où l'agent tensioactif est poloxamine.

5. Solution multifonctionelle selon l'une quelconque des revendications précédentes, où l'agent antimicrobien est polyhexaméthylène biguanide, PHMB.

6. Solution multifonctionelle selon l'une quelconque des revendications précédentes, où l'agent chélatant est l'édétate disodique, Na₂-EDTA.

7. Solution multifonctionelle selon l'une quelconque des revendications précédentes, où l'agent de tonicité est le chlorure de sodium et/ou le sorbitol.

8. Solution multifonctionelle selon l'une quelconque des revendications précédentes, où le composant acide du système tampon est l'acide borique.

9. Solution multifonctionelle selon l'une quelconque des revendications 1 jusqu'à 8, où l'agent de nettoyage et composant alcalin du système tampon est le citrate de sodium.

10. Solution multifonctionelle selon la revendication 9, avec la composition suivante:
| **Ingrédient** | **mg/ml** |
|---|---|
| Poloxamine 1107 | 10,0 |
| Acide borique | 6,4 |
| Citrate de sodium | 6,0 |
| Édétate disodique (Na₂-EDTA) | 1,0 |
| Extrait de camomille | 0,025 |
| Polyhexaméthylène biguanide (PHMB) | 0,001 |
| Sorbitol | 10,0 |
| Chlorure de sodium (NaCl) | 3,5 |
| NaOH / HCl | q.s. pour pH = 7,0-7,6 |

11. Solution multifonctionelle selon l'une quelconque des revendications 1 jusqu'à 8, où l'agent de nettoyage et composant alcalin du système tampon est un mélange de phosphate monobasique de sodium, NaH₂PO₄, et de phosphate dibasique de sodium, Na₂HPO₄.

12. Solution multifonctionelle selon la revendication 11, avec la composition suivante:
| **Ingrédient** | **mg/ml** |
|---|---|
| Poloxamine 1107 | 10,0 |
| Acide borique | 5,0 |
| Na₂HPO₄ | 2,0 |
| NaH₂PO₄ | 0,08 |
| Édétate disodique (Na₂-EDTA) | 1,0 |
| Extrait de camomille | 0,025 |
| Polyhexaméthylène biguanide (PHMB) | 0,001 |
| Chlorure de sodium (NaCl) | 6,0 |
| NaOH / HCl | q.s. pour pH = 7,0-7,6 |
